# EUROPEAN PATENT APPLICATION

(11) **EP 4 759 262 A1**
(43) Date of publication of application: **17.06.2026**
(21) Application number: 24218755.7
(22) Date of filing: 10.12.2024
(51) Int. Cl.: A61B 90/00, A61B 6/04, A61B 17/00, A61B 34/20, A61B 5/00

(54) **SENSOR APPARATUS WITH FIDUCIAL MARKERS AND METHOD FOR IDENTIFICATION THEREOF IN IMAGE DATA**

(71) Applicant: Koninklijke Philips N.V., 5656 AE Eindhoven (NL)
(72) Inventor: WIEMKER, Rafael, Eindhoven (NL); KRUEGER, Sascha, Eindhoven (NL); SENEGAS, Julien Thomas, 5656AG Eindhoven (NL); VAN EE, Raymond, Eindhoven (NL); WIRTZ, Daniel, Eindhoven (NL); DENISSEN, Adrianus Johannes Maria, Eindhoven (NL)
(74) Representative: Philips Intellectual Property & Standards

(57) **Abstract**

It would be desirable to provide a sensor apparatus that facilitates precise mapping of signals to sensor nodes in an image coordinate system. There is therefore provided a sensor apparatus (110) comprising a plurality of sensor nodes. The sensor apparatus is configured to be used in conjunction with an imaging device (104). The imaging device is configured to acquire image data, wherein the image data uses an image coordinate system. The sensor apparatus comprises a plurality of fiducial markers each having a uniquely identifiable pattern which is detectable by the imaging device. The sensor apparatus is configured to hold the fiducial markers in a predetermined spatial relationship with the sensor nodes for mapping of signals obtained from the sensor nodes to locations of the sensor nodes in the image coordinate system.

## Description

### FIELD OF THE INVENTION

The present invention relates generally to sensor technology and more specifically to a sensor apparatus equipped with fiducial markers that enable mapping of sensor signals to sensor node locations within a coordinate system of an imaging system, and to a corresponding method for detecting and mapping fiducial markers to sensor node locations.

### BACKGROUND OF THE INVENTION

In various imaging fields, including medical imaging, there is a need to accurately map sensor signals to spatial locations of sensors nodes in a defined coordinate system. Traditional sensor apparatus often lacks capability for precise spatial referencing, limiting its usefulness in applications requiring accurate alignment between sensor data and spatial images. Existing solutions enabling precise spatial referencing may be complex or limited in adaptability.

### SUMMARY OF THE INVENTION

It would be desirable to provide a sensor apparatus that facilitates precise mapping of signals to sensor nodes in an image coordinate system.

To better address one or more of these concerns, there is provided, in a first aspect of invention, a sensor apparatus comprising a plurality of sensor nodes, wherein the sensor apparatus is configured to be used in conjunction with an imaging device, wherein the imaging device is configured to acquire image data, wherein the image data uses an image coordinate system, the sensor apparatus comprising a plurality of fiducial markers each having a uniquely identifiable pattern which is detectable by the imaging device, wherein the sensor apparatus is configured to hold the fiducial markers in a predetermined spatial relationship with the sensor nodes for mapping of signals obtained from the sensor nodes to locations of the sensor nodes in the image coordinate system.

### Sensor apparatus and sensor nodes

In an example, the sensor apparatus is configured as a sensing mattress, and the sensor nodes are arranged as an array of sensor nodes contained within the sensing mattress. The sensor nodes of the array may be configured to measure one or more physical quantities, e.g., pressure, temperature, electrical field (EEG), magnetic field (MEG). The sensing mechanism for a particular physical quantity may comprise one or more of: fiber optical (advantageous for MR- and CT imaging compatibility); electrical; piezoelectrical; quantum-magnetic (e.g., SQUIDs); gas bellow sensors; capacitive. The sensor nodes of the array may be of the same kind, or of mixed type. In an example the sensing mattress comprises a pressure-sensing array (such as an optical waveguide pressure-sensing array), which comprises a plurality of intersecting optical fibers forming the array, with each intersection serving as a pressure sensor node. Numerous array configurations are conceivable, exhibiting various shapes and arrangements, such as rectangular or non-rectangular arrays, the latter comprising e.g. circular or hexagonal arrays.

### Imaging device

The imaging device may comprise a medical imaging device. The imaging device may comprise a volumetric (3D) imaging device. The imaging device may comprise an imaging scanner. The imaging device may comprise a magnetic resonance imaging (MRI) scanner, a computed tomography (CT) scanner, or a hybrid scanner such as PET-CT or PET-MR.

### Image data

The image data may comprise raw/quantitative data, image data constructed from the raw data, and/or labelling data. The image data uses an image coordinate system, meaning that spatial locations are describable in the image data in terms of the image coordinate system. In the case that the imaging device comprises a volumetric imaging device, the image data may comprise an image volume.

### Fiducial markers

The fiducial markers are discernible in the imaging modality (for example, by virtue of their radiopacity for CT, or proton-density for MRI). Preferably, the fiducial markers do not cause imaging artefacts (for example, by virtue of their being non-metallic). The fiducial markers are furthermore transparent to the sensor-measuring quantity (e.g., pressure, temperature, etc.).

In an example, the fiducial markers are configured as attachments to the sensing mattress, whether removably or non-removably attached. In an example, the fiducial markers are enclosed within the sensing mattress. The fiducial markers may be at least partially 3D-printed.

### Uniquely identifiable pattern

The uniquely identifiable pattern of each fiducial marker may be encoded using a predetermined coding scheme. The uniquely identifiable pattern of each fiducial marker may be encoded using at least one of the following: binary hole code; bar code; QR code; margin pattern. Additionally or alternatively, alphanumeric or color-based patterns may be used.

The uniquely identifiable pattern of each fiducial marker may be formed via addition, removal, or change of material in the respective fiducial marker. It will be appreciated that additional ways to create the pattern in the fiducial markers may be employed, such as application of coatings, layering of materials, or embedding of contrast materials.

### Predetermined spatial relationship

The spatial relationship between fiducial markers and sensor nodes may be fixed or dynamically adjustable. The spatial relationship enables the unknown location of each sensor node to be determined with respect to the detectable location of its respective fiducial marker.

Mapping of signals obtained from the sensor nodes to locations of the sensor nodes in the image coordinate system will now be described with reference to a second aspect.

In the second aspect, there is provided a computer-implemented method for identification of sensor nodes in image data, the method comprising:
obtaining image data from an imaging device, wherein the imaging device is configured to acquire image data, wherein the image data uses an image coordinate system;
obtaining a plurality of sensor signals from a sensor apparatus used in conjunction with the imaging device, the sensor apparatus comprising a plurality of sensor nodes and a plurality of fiducial markers each having a uniquely identifiable pattern which is detectable by the imaging device, wherein the sensor apparatus is configured to hold the fiducial markers in a predetermined spatial relationship with the sensor nodes;
detecting and identifying the fiducial markers in the image data using the uniquely identifiable pattern of each fiducial marker;
determining locations of the sensor nodes in the image coordinate system based on the detected and identified fiducial markers; and
mapping the sensor signals obtained from the sensor nodes to the determined locations of the sensor nodes.

The method may further comprise searching the image data utilizing a pattern matching algorithm to detect and identify the fiducial markers in the image data.
The method may further comprise preprocessing the image data to enhance contrast before applying the pattern matching algorithm to detect and identify the fiducial markers.

The detection and identification of the fiducial markers may comprise applying a machine learning-based image recognition algorithm as the pattern matching algorithm. In the case that the image data comprises raw image data, the pattern matching algorithm may be applied to the raw image data to detect and identify the fiducial markers. In the case that the image data comprises an image which has been constructed from raw image data, the pattern matching algorithm may be applied to the image to detect and identify the fiducial markers.

Transmission of the signals by the sensor nodes may comprise at least one of wireless (e.g., Bluetooth, Wi-Fi), wired, and optical data transfer.

According to a third aspect, there is provided a computing system configured to perform the method of the second aspect.

According to a fourth aspect, there is provided a computer program (product) comprising instructions which, when executed by a computing system, enable or cause the computing system to perform the method of the second aspect.

According to a fifth aspect, there is provided a computer-readable (storage) medium comprising instructions which, when executed by a computing system, enable or cause the computing system to perform the method of the second aspect. The computer-readable medium may be transitory or non-transitory, volatile or non-volatile.

The computing system can typically comprise a processor, for example a processor that is part of a computer.

The invention provides a sensor apparatus comprising a plurality of sensor nodes and fiducial markers, each with a uniquely identifiable pattern, facilitating the mapping of sensor node signals to locations within the image coordinate system. The fiducial markers are positioned in a predetermined spatial relationship with the sensor nodes, allowing a computing system to identify their positions within image data generated by the imaging device.

The term "obtaining", as used herein, may encompass receiving from another system, device, or process; receiving via an interaction with a user; loading or retrieving from storage or memory; measuring or capturing using sensors or other data acquisition circuitry.

The term "determining", as used herein, may encompass calculating, computing, processing, deriving, investigating, resolving, selecting, choosing, ascertaining, establishing, looking up (e.g., looking up in a table, a database or another data structure), receiving (e.g., receiving information), accessing (e.g., accessing data in a memory), and the like.

The indefinite article "a" or "an" does not exclude a plurality. In addition, the articles "a" and "an" as used herein should generally be construed to mean "one or more" unless specified otherwise or clear from the context to be directed to a singular form.

Unless specified otherwise, or clear from the context, the phrases "one or more of A, B and C", "at least one of A, B, and C", and "A, B and/or C" as used herein are intended to mean all possible permutations of one or more of the listed items. That is, the phrase "A and/or B" means (A), (B), or (A and B), while the phrase "A, B, and/or C" means (A), (B), (C), (A and B), (A and C), (B and C), or (A, B, and C).

The term "comprising" does not exclude other elements or steps. Furthermore, the terms "comprising", "including", "having" and the like may be used interchangeably herein.

The invention may include one or more aspects, examples or features in isolation or combination whether specifically disclosed in that combination or in isolation. Any optional feature or sub-aspect of one of the above aspects applies as appropriate to any of the other aspects.

The above-described aspects will become apparent from, and elucidated with, reference to the detailed description provided hereinafter.

### BRIEF DESCRIPTION OF THE DRAWINGS

A detailed description will now be given, by way of example only, with reference to the accompanying drawings, in which:-
Fig. 1 illustrates a medical imaging device used in conjunction with a sensor apparatus comprising fiducial markers;
Fig. 2 is a diagram showing fiducial marker encoding options, including binary hole codes, bar codes, margin patterns, and QR codes; and
Fig. 3 is a flowchart illustrating a computer-implemented method for detecting and mapping fiducial markers to sensor node locations.

### DETAILED DESCRIPTION OF EMBODIMENTS

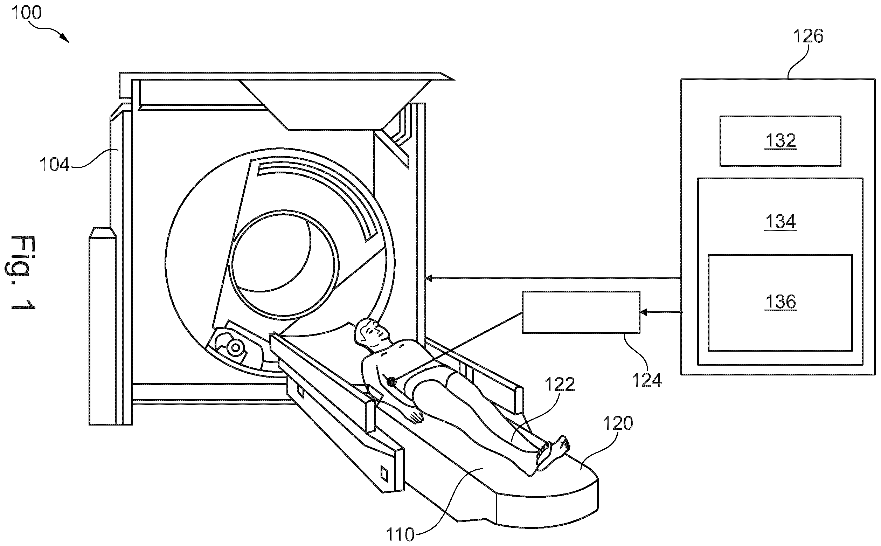

Fig. 1 illustrates a system 100 according to the present disclosure.

The system 100 comprises a medical imaging device 104, which in this example is a volumetric imaging device (e.g. CT, MR). The medical imaging device 104 is configured to carry out a medical imaging procedure for generating an image volume depicting at least part of a patient 122. The medical imaging procedure may comprise a scan sequence for generating the image volume. The patient 122 is presented to the medical imaging device 104 by way of a patient support 120, which may be movable.

A sensor apparatus 110 is arranged to obtain signals at a plurality of body locations of the patient 120. In this non-limiting example, the sensor apparatus 110 comprises an optical waveguide pressure sensing array such as that described in WO 2017/144675 A1, configured as a sensing mattress. The sensing mattress 110 comprises a plurality of sensor nodes arranged in a spatial configuration, such as an array. The spatial configuration may be rectangular, circular, or hexagonal, according to different applications and user needs. The sensor nodes can be embedded within or attached to the sensing mattress 110. In the latter case, the sensor nodes may be permanently or removably attached, with removability enabling flexibility in maintenance and replacement.

The patient 122 may be positioned at an arbitrary location with respect to the sensing mattress 110. For example, the sensing mattress 110 may be placed underneath, on top of, or wrapped around the patient 122. Moreover, the sensing mattress 110 may be placed in different positions and orientations with respect to the medical imaging device 104, while varying in configuration and size across different models in a range. The sensing mattress 110 may be disposable for hygienic reasons. Additionally, patient body size, shape and posture may vary arbitrarily.

When used in conjunction with the medical imaging device 104, it is beneficial for the signal analysis to have precise location information for each sensor node in the coordinate system of the image volume. This spatial relationship is not trivial to establish when the sensing mattress 110 is used as a non-scanner-mounted, free-floating apparatus in the above-described manner.

Described herein is a sensing mattress 110 comprising fiducial markers, each having a uniquely identifiable pattern which is detectable by the imaging device 104, attached to the sensor nodes. The sensing mattress 110 is configured to hold the fiducial markers in a predetermined spatial relationship with the sensor nodes. The uniquely identifiable patterns enable signal post-processing for precise mapping of signals obtained from the sensor nodes with respect to the locations of the sensor nodes in the image volume. The use of such a sensing mattress 110 is of particular benefit inside the gantry of the medical imaging device 104, e.g. on the patient support 120, such that the sensor signals obtained from the sensing mattress 110 can be interpreted in conjunction with the image volume, yielding important medical insights.

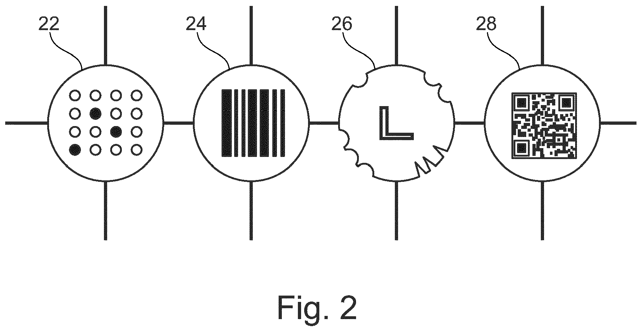

Fig. 2 is a diagram showing fiducial marker encoding options. Fiducial marker 22 uses a binary hole code pattern (e.g., a punch card pattern). Fiducial marker 24 uses a bar code pattern (reeve pattern). Fiducial marker 26 uses a margin pattern (intruding from the fringe). Fiducial marker 28 uses a QR code pattern. The patterns are detectable in the image volume acquired by the imaging device 104. Alternatively, the patterns may be detectable in the raw signals (e.g., MR readouts or CT projections). The fiducial markers may be made to exhibit the uniquely identifiable pattern via material removal, addition, or contrast enhancement, for example via formation of holes, material thinning (e.g., reeves), or material changes (e.g., solid inserts or liquid fillings with for example a contrast agent fluid). The material whereby the pattern is formed, hereinafter the "decorator material", is chosen to be transparent to the sensor-measuring quantity (e.g., pressure, temperature, etc.) but to be discernible in the imaging modality (e.g., via radiopacity for CT, proton density for MR). Preferably, the fiducial markers cause no imaging artefacts, such as streaking artefacts, beam-hardening, etc., by virtue of their being non-metallic. More particularly, the decorator material may be visible in the modality only within a given frequency range (for MR, for example, using decorator materials with predefined resonance frequencies like fluor for MR) or may be associated with unique absorption values (e.g. Hounsfield units for CT). This has the advantage of making the decorator material easily and uniquely distinguishable from other materials. This may also have the further advantage that the pattern is not visible in regular patient images, if desired. The patterns may furthermore be hidden from view by the naked eye, for example in the case that the fiducial markers are covered by an outer enveloping layer of the sensing mattress 110.

Manufacturing of the fiducial markers may be carried out using printable or layerable materials, for example using digital 3D-printing, as individual patterns are required and mass-production-molds are costly.

The sensing mattress 110 holds the fiducial markers in a predetermined spatial relationship with the sensor nodes, to facilitate mapping of signals obtained from the sensor nodes to locations of the sensor nodes in the image coordinate system. The relative position of each fiducial marker pattern to its respective sensor node is determined precisely by construction/design or by calibration after manufacturing.

In this way, signals obtained from each mattress-internal sensor node may be put into a precise spatial relationship with the body-internal organs of interest manifesting in the image volume, allowing mapping of signal propagation patterns, and correction of propagation runtime confounders (time delays between signal time-curves and organs).

Referring back to Fig. 1, the system 100 further comprises a computing system 126 which is configured to obtain sensor signals from the sensing mattress 110 along with image data from the imaging device 104. The computing system 126 comprises a processor 132 and a computer-readable storage medium 134 storing instructions 136 for carrying out a method for detecting and mapping fiducial markers to sensor node locations as described herein.

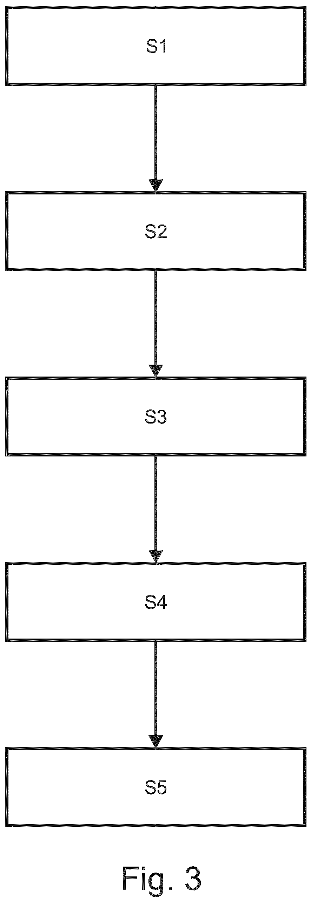

Fig. 3 is a flowchart illustrating the computer-implemented method for detecting and mapping fiducial markers to sensor node locations. The method comprises the following steps.

### S1: Data Acquisition

Data acquisition step S1 comprises obtaining image data from an imaging device, wherein the imaging device is configured to acquire image data, wherein the image data uses an image coordinate system. Data acquisition step S1 further comprises obtaining a plurality of sensor signals from a sensor apparatus used in conjunction with the imaging device, the sensor apparatus comprising a plurality of sensor nodes and a plurality of fiducial markers each having a uniquely identifiable pattern which is detectable by the imaging device, wherein the sensor apparatus is configured to hold the fiducial markers in a predetermined spatial relationship with the sensor nodes. For example, the imaging device 104, such as an MRI system, or a camera, captures an image of the sensor apparatus 110 with fiducial markers detectable in the image data. Simultaneously, the sensor nodes acquire sensor signals corresponding to environmental or physiological parameters (e.g., pressure, temperature).

### S2: Pattern Detection and Identification

Pattern detection and identification step S2 comprises detecting and identifying the fiducial markers in the image data using the uniquely identifiable pattern of each fiducial marker. The computing system processes the image data to detect and identify each fiducial marker based on its unique pattern. This may involve a pattern-matching algorithm being applied to the raw or preprocessed image data. Optional preprocessing, such as contrast enhancement, may be used to improve detection accuracy, especially in low-contrast environments. The detection and identification of the patterns can occur in images acquired by the imaging system or in dedicated raw data.

In the case of detection in images, two options are envisaged by the present disclosure. In the first option, a dedicated imaging acquisition is performed, for example frequency or spatially selective. Since the sensing mattress 110 is located between the patient support 120 and the patient 122, only the volume around the patient support 120 is imaged. Using frequency selective pulses, only images of the pattern material can be generated. In the second option, the patterns are imaged together with the patient 122, which has the advantage that no additional scan is needed. In this case, it may be necessary to extend the field of view of the acquisition so as to include the patient support 120. Subsequently, the image volume generated by the scanner is automatically searched exhaustively using pattern matching algorithms for the known patterns, and the location of each sensor node is thus established in the image coordinate system.

In the case that the detection of the pattern is done directly in raw signals, it may be advantageous to acquire multiple spatially selective data. Then, for each of the acquired raw signals, an algorithm detects whether the characteristic signature of the pattern is present or not.

### S3: Coordinate Determination

Coordinate determination step S3 comprises determining locations of the sensor nodes in the image coordinate system based on the detected and identified fiducial markers. Once identified, the fiducial markers provide spatial reference points within the imaging coordinate system. The computing system maps these reference points to the physical layout of the sensor nodes within the sensor apparatus 110, using the predetermined spatial relationship between fiducial markers and sensor nodes.

### S4: Signal Mapping

Signal mapping step S4 comprises mapping the sensor signals obtained from the sensor nodes to the determined locations of the sensor nodes. The computing system correlates sensor signals from each sensor node to its spatial location within the image coordinate system. This enables accurate mapping of the sensor signals in the spatial context defined by the image.

### S5: Output and Display

Output and display step S5 comprises outputting the mapped sensor signals. The spatially mapped sensor data is then outputted in a form that aligns sensor signals with specific regions in the image data. This can be displayed or further processed, depending on the application requirements.

### Example 1: Medical Imaging Application

In a medical imaging context, the sensor apparatus 110 may be configured as a sensing mattress designed to monitor pressure distribution for a patient undergoing MRI. The sensing 110 mattress includes a grid of pressure-sensitive sensor nodes and fiducial markers 28 with QR codes. The MRI system 104 captures images including the sensing mattress 110, which are processed by a computing system to identify each fiducial marker 28, locate the corresponding sensor node, and map the pressure data onto the image. This enables real-time visualization of pressure distribution during imaging.

### Example 2: Robotic Guidance System

In a robotic guidance application, the sensor apparatus 110 is attached to a flexible mat with embedded temperature sensors and fiducial markers encoded with color patterns. A camera system observes the mat, identifying the fiducial markers, and the computing system maps temperature data to precise mat coordinates. The robotic system uses this data to adjust its movements based on temperature changes detected across the mat, providing a spatially aware temperature map for navigation.

### Example 3: Augmented Reality Interface

For an augmented reality (AR) interface, the sensor apparatus 110 includes removable sensor nodes placed on a wearable garment, with fiducial markers in alphanumeric codes applied by 3D printing. A camera-based imaging system identifies these markers, mapping their positions to the garment layout. The computing system processes pressure sensor data, creating a real-time AR overlay that displays pressure distribution on the user's body, enabling enhanced feedback for training or therapy.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

It has to be noted that embodiments of the invention are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The mere fact that certain measures are recited in mutually different dependent claims does not indicate that a combination of these measures cannot be used advantageously.

Any reference signs in the claims should not be construed as limiting the scope.

## Claims

1. A sensor apparatus comprising a plurality of sensor nodes, wherein the sensor apparatus is configured to be used in conjunction with an imaging device, wherein the imaging device is configured to acquire image data, wherein the image data uses an image coordinate system, the sensor apparatus comprising a plurality of fiducial markers each having a uniquely identifiable pattern which is detectable by the imaging device, wherein the sensor apparatus is configured to hold the fiducial markers in a predetermined spatial relationship with the sensor nodes for mapping of signals obtained from the sensor nodes to locations of the sensor nodes in the image coordinate system.

2. The sensor apparatus of claim 1, wherein the uniquely identifiable pattern of each fiducial marker is encoded using at least one of the following: binary hole code; bar code; QR code; margin pattern.

3. The sensor apparatus of claim 1 or 2, wherein the uniquely identifiable pattern of each fiducial marker is formed via addition, removal, or change of material in the respective fiducial marker.

4. The sensor apparatus as claimed in any preceding claim, wherein the sensor apparatus is configured as a sensing mattress, and wherein the sensor nodes are arranged as an array of sensor nodes contained within the sensing mattress.

5. The sensor apparatus as claimed in claim 4, wherein the fiducial markers are configured as attachments to the sensing mattress.

6. The sensor apparatus as claimed in claim 4, wherein the fiducial markers are enclosed within the sensing mattress.

7. The sensor apparatus as claimed in any preceding claim, wherein the fiducial markers are at least partially 3D-printed.

8. A computer-implemented method for identification of sensor nodes in image data, the method comprising:
obtaining image data from an imaging device, wherein the imaging device is configured to acquire image data, wherein the image data uses an image coordinate system;
obtaining a plurality of sensor signals from a sensor apparatus used in conjunction with the imaging device, the sensor apparatus comprising a plurality of sensor nodes and a plurality of fiducial markers each having a uniquely identifiable pattern which is detectable by the imaging device, wherein the sensor apparatus is configured to hold the fiducial markers in a predetermined spatial relationship with the sensor nodes;
detecting and identifying the fiducial markers in the image data using the uniquely identifiable pattern of each fiducial marker;
determining locations of the sensor nodes in the image coordinate system based on the detected and identified fiducial markers; and
mapping the sensor signals obtained from the sensor nodes to the determined locations of the sensor nodes.

9. The method as claimed in claim 8, further comprising searching the image data utilizing a pattern matching algorithm to detect and identify the fiducial markers in the image data.

10. The method as claimed in claim 9, wherein the image data comprises raw image data, and wherein the pattern matching algorithm is applied to the raw image data to detect and identify the fiducial markers.

11. The method as claimed in claim 9, wherein the image data comprises an image which has been constructed from raw image data, and wherein the pattern matching algorithm is applied to the image to detect and identify the fiducial markers.

12. A computing system configured to perform the method as claimed in any of claims 1-11.

13. A computer-readable medium comprising instructions which, when executed by a computing system, cause the computing system to perform the method as claimed in any of claims 1-11.
